# EUROPEAN PATENT APPLICATION

(11) **EP 0 808 613 A1**
(43) Date of publication of application: **26.11.1997**
(21) Application number: 97201453.4
(22) Date of filing: 14.05.1997
(51) Int. Cl.: A61F 2/06

(54) **Tubular prosthesis made of curable material**

(30) Priority: 21.05.1996 NL 1003178
(71) Applicant: CORDIS EUROPA N.V., NL-9301 LJ Roden (NL)
(72) Inventor: Ligtenberg, Hendrikus Cornelis Geert, 9351 PX Leek (NL); Haan, Marcel Gerhard, 9301 EA Roden (NL)
(74) Representative: 't Jong, Bastiaan Jacobus

(57) **Abstract**

This invention relates to a tubular prosthesis comprising a substantially tubular basic body made of material expandable in at least the radial direction, which has been provided with at least one ridge extending around the prosthesis made of extensible and curable material. In addition the ridge may comprise reinforcing fibres extending in the longitudinal direction of the ridge.

## Description

The invention relates to a tubular prosthesis as known for instance from the Dutch patent application 9 101 159.

Such a tubular prosthesis is used for instance as a so-called stent for supporting a blood vessel internally, following angioplastic treatment of a narrowing.

The object of the invention is to further improve the known tubular prosthesis. This is achieved with the measures as characterised in claim 1.

Due to the ridge extending around the prosthesis, the required annular strength, that is to say the resistance to compression of the prosthesis, is achieved, whilst at the same time the prosthesis is pliable in axial direction. As a result the prosthesis can be employed in situations in which pliability of the prosthesis is a physiological necessity, like for instance due to the movement of organs et cetera.

A very advantageous embodiment has additionally been characterised in claim 2. With a relatively small cross-section the ridges can be given a very large bending strength as a result of which the annular strength of the prosthesis will be very large.

By employing the measure as set out in claim 3 the prosthesis remains in the non-cured state of the curable material very movable, in particular expandable. After curing of the curable material the ridges form a stiff element of the prosthesis extending around the basic body.

Preferably the measure as set out in claim 4 is employed. By means of a catheter provided with a light conductor the prosthesis can in that case be arranged in a required position inside the body of a patient.

By employing the measure as set out in claim 5 the outer surface acquires to a certain degree a ridged surface, as a result of which a very good engagement of the prosthesis in the wall of the body vessel inside of which it has been arranged is achieved.

The pliability of the prosthesis in a longitudinal direction can be varied by employing the measure as set out in claim 6. There where the reinforcing ridges or parts thereof are positioned close to each other a high bending stiffness is obtained, whilst in sections in which the reinforcing ridges or parts thereof are placed at a greater distance from one another, a greater pliability is obtained. Consequently the prosthesis according to the invention can be made to suit specific applications.

A suitable embodiment in this respect is characterised in claim 7. Due to the greater stiffness at the ends, good engagement of the prosthesis is ensured.

With the measure as set out in claim 8 a maximal annular strength of the prosthesis is achieved with a minimal cross-section of the ridge.

The preferred embodiment of the invention characterised in claim 9 has the advantageous property that it is very expandable.

The invention will be explained in greater detail in the following description with reference to the attached figures.

Figure 1 illustrates a tubular prosthesis according to the invention arranged around a balloon catheter, of which only the end-section has been shown.

Figure 2 shows a view corresponding to figure 1, in which case the balloon member together with the tubular prosthesis arranged around it has been expanded.

Figure 3 shows a detailed view along the line III in figure 2.

Figures 4 and 5 show variations of embodiments corresponding to figure 3.

Figure 6 shows a perspective view of a prosthesis according to the invention with annular reinforcing ridges.

Figure 7 shows a view corresponding to figure 6 of a prosthesis according to the invention with a helically shaped ridge.

Figure 8 shows a view corresponding to figures 6 and 7 of a prosthesis according to the invention with once again differently shaped ridges.

Figure 9 shows a side view of a prosthesis according to the invention with crossing helically shaped ridges.

Figure 10 shows an embodiment partly corresponding to figure 9, which in addition has been provided with annular ridges.

Figure 11 shows a side view of a prosthesis according to the invention with annular ridges placed at a varying distance from one another.

Figure 12 shows yet another variation of an embodiment.

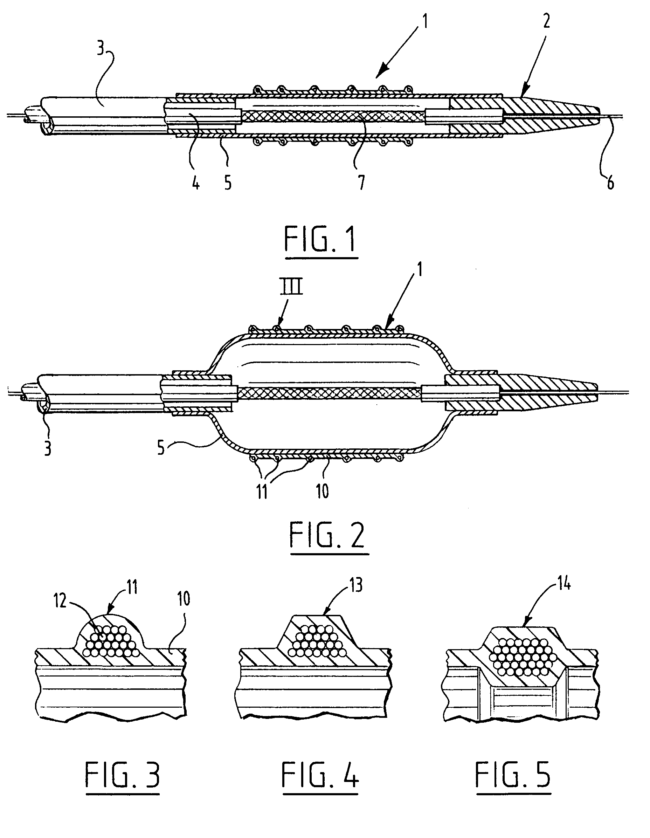

Figure 1 shows a tubular prosthesis 1 according to the invention in non-expanded state, in which it can be introduced into a patient. The tubular prosthesis 1 shown here is a stent and has been designed for the purpose of supporting a blood vessel at a site where a narrowing has been removed by means of angioplasty.

The prosthesis 1 has been arranged in a manner known as such around a balloon member 5 of the catheter 2, which furthermore comprises an outer tube-like body 3 and an inner tube-like body 4 extending through a lumen thereof. The inner tube-like body 4 also comprises a lumen through which in this example a guide wire 6 extends.

In the inner tube-like body 4 a light conductor has been integrated which is uncovered in the section 7 extending inside the balloon 5. Due to this light conductor, light can be conducted from the proximal end of the catheter 2, which remains outside the body of the patient, to the section 7 where it can be emitted radially.

In particular UV light is conducted via the light conductor, used for curing the material of which the ridge 11 has been made, which in that case will comprise a plastic material curing under the influence of UV light.

Figure 2 shows the catheter with the prosthesis 1 of figure 1 in the situation in which the balloon member 5 has been expanded by supplying a medium, in particular a liquid under pressure via the central Lumen of the outer tube-like body 3. Due to the expansion of the balloon member 5 also the prosthesis 1 is expanded so that it supports the blood vessel with the required passage not shown here .

The prosthesis 1 according to the invention comprises a basic body 10 made of a pliable material. In addition the prosthesis 1 comprises ridges 11 which provide the prosthesis 1 with a relatively large annular strength, that is to say a large resistance to compression of the prosthesis 1.

Figure 3 illustrates in greater detail the cross-section of the ridge 11. As has been illustrated, the ridge 11 comprises in this case a number of reinforcing fibres 12 which have been embedded in the curable material.

Following expansion in the manner shown in figure 2, the curable material receiving the fibres 12 can be cured by exposing this material to UV light.

As can be seen in the figures, the ridges 11 protrude partially from the surface of the basic body 10. Consequently, the outer surface of the prosthesis 1 has a ridged or ribbed appearance, as a result of which a good grip of the prosthesis 1 on the wall of the blood vessel is obtained. Shifting of the prostheses 1 in the longitudinal direction will consequently be prevented in a reliable manner.

With the embodiment of figure 4 the ridge 13 has been given an angular cross-section, as a result of which an even better grip on the wall of the vessel can be obtained.

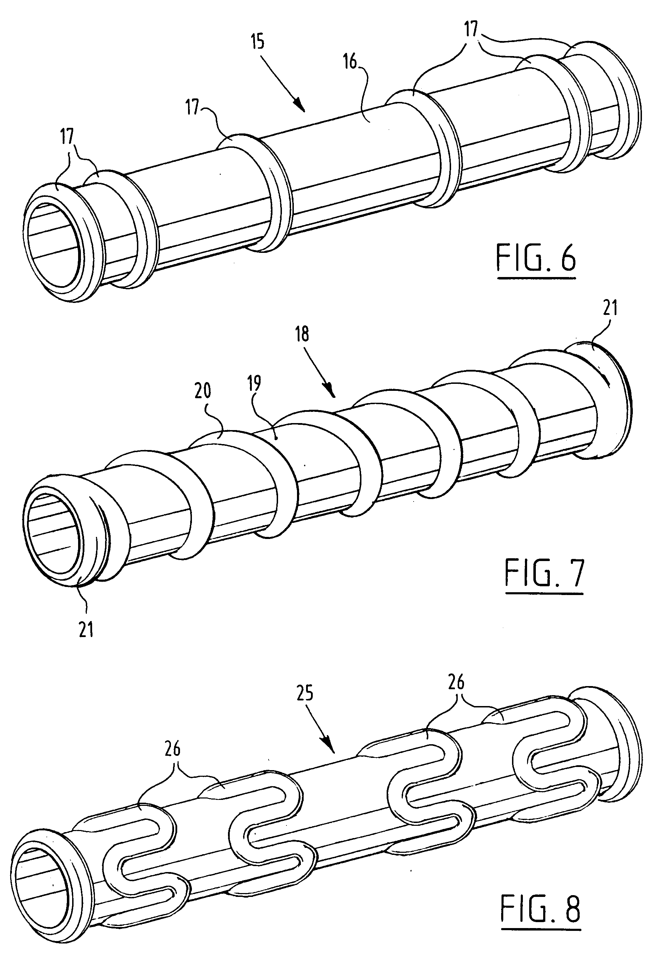

The ridge 14 as shown in figure 5 partially extends outside the outer surface of the basic body and partially inside. As a result a ridge 14 with a relatively large cross-section and consequently a relatively large stiffness can be formed, without the outer wall of the prosthesis showing too large differences in height.

Figure 6 shows a tubular prosthesis 15 according to the invention, whereby the tubular basic body 16 with the annular ridges 17 extending around the basic body 16 can be seen clearly. With this embodiment, in which case the ridges 17 are annular, a maximum annular stiffness is achieved. In the case the ridges 17 comprise reinforcing fibres the prosthesis 15 can be expanded less easily however. A better expandability can in this case be achieved by making the reinforcing fibres relatively short, as a result of which they can move past each other during expansion.

With the embodiment of figure 7 the prosthesis 18 comprises a ridge 20 extending in a helical line along the basic body 19. A prosthesis 18 made in this way can expand more easily, for instance due to the fact that the ends can twist a little in relation to one another. As illustrated, the helically shaped ridge 20 forms annular ridges 21 at the ends, so that a good stiffness against compression is achieved at these ends.

The tubular prosthesis 25 according to the invention illustrated in figure 8 comprises in the circumferential direction zigzag-shaped ridges 26 which can be made to extend well. In the expanded state the zigzag-strokes are straightened, as a result of which they provide good annular stiffness in the cured state. The basic body has been made of a material which is pliable to such an extent that the movements caused by stretching the reinforcing ridges 26 can be absorbed in the material whereby the basic body unfolds.

It will be clear that with the prosthesis according to the invention, with a suitable choice of the shape of the ridge a required annular strength and a required axial bending strength of the prosthesis can be obtained. Also the choice of material can contribute to the required properties. As a result the required high resistance to compression is obtained on the one hand, whilst on the other hand the prosthesis is pliable in the longitudinal direction in order to adapt to movements of the body vessel, in particular the blood vessel inside of which the prosthesis has been received.

Depending on the intended use, all known fibres are suitable by way of reinforcing fibres for the ridges. Glass fibres or quartz fibres, but also synthetic fibres made of for instance nylon, polycarbonate, polyethene terephthalate or polyester can be used. In certain cases also aramide or carbon fibres can be used. In order to achieve good flexibility in the radial direction, the fibres inside the ridges themselves can extend in a helical pattern. Also relatively short fibres can be used so that they can move past each on expansion in order to achieve the required radial extension. Apart from directional fibres also non-directional fibres can be used like in non-braided fibrous material.

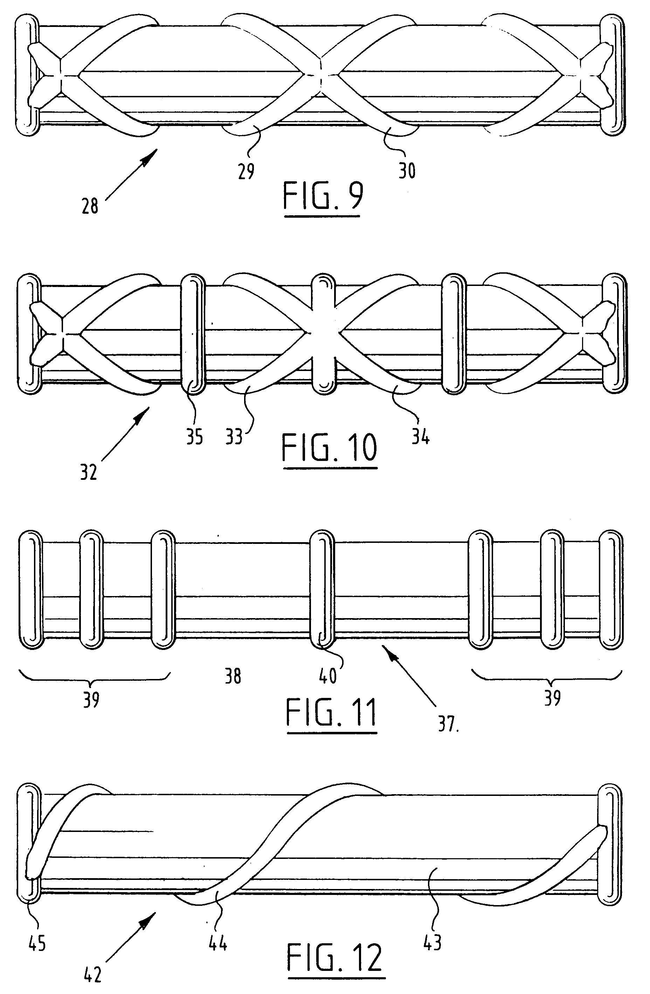

The figures 9 - 12 show further variations of embodiments. With the prosthesis 28 of figure 9 for instance the two helically shaped ridges 29, 30 have been employed, which cross each other in several places.

Figure 10 is a configuration whereby the prosthesis 32 comprises helically shaped ridges 33 and 34 crossing each other, to which a number of annular reinforcing ridges 35 have been added.

With the prosthesis 37 according to figure 11 the ridges 39 close to the ends have been arranged to the basic body at a relatively small distance form one another. In the space in between the groups of reinforcing ridges 39 additionally one ridge 40 has been arranged, which consequently has a greater distance to the adjacent ridge. In this way the bending stiffness of the prosthesis 37 is adjusted in a required manner in the longitudinal direction. Close to the ends, where the ridges are placed relatively close to one another, a large bending stiffness is achieved, whilst in the intervening area less bending stiffness and consequently more pliability of the prosthesis 37 is achieved.

Finally, figure 12 shows a prosthesis 42 with a basic body 43 around which one single helically shaped ridge 44 extends, which ends in an annular ridge 45 at the ends. In the cured state of the ridge 44 it forms as it were a helical spring which allows a very great pliability of the basic body 43, whilst at the same time it provides a significant resistance to compression.

The prosthesis according to the invention can be realized in a great diversity of embodiments for the purpose of a great number of applications. The invention is consequently not limited to the embodiments illustrated in the figures, which have only been given by way of illustration of the invention.

## Claims

1. Tubular prosthesis comprising a substantially tubular basic body made of a material expandable at least in the radial direction, which has been provided with at least one ridge extending around the prosthesis made of extensible and curable material.

2. Tubular prosthesis as claimed in claim 1, wherein the ridge comprises reinforcing fibres extending in the longitudinal direction of the ridge.

3. Tubular prosthesis as claimed in claim 2, wherein the reinforcing fibres have been embedded in curable material.

4. Tubular prosthesis as claimed in one of the previous claims, wherein the curable material cures due to exposure to radiation, such as UV light.

5. Tubular prosthesis as claimed in one of the previous claims, wherein the ridge protrudes at least partially in relation to the outer surface of the basic body.

6. Tubular prosthesis as claimed in one of the previous claims, wherein the mutual axial distance of sections of the ridge extending around the prosthesis or ridges extending around the prosthesis respectively, varies along the length of the basic body.

7. Tubular prosthesis as claimed in claim 6, wherein the mutual axial distance close to the ends of the basic body is smaller than in the intervening area.

8. Tubular prosthesis as claimed in one of the previous claims, comprising a number of annular ridges.

9. Tubular prosthesis as claimed in one of the previous claims, comprising at least one ridge extending in a helical pattern.
